# FASCICULE DE BREVET EUROPEEN

(11) **EP 3 509 659 B1**
(45) Date de publication et mention de la délivrance du brevet: **08.07.2020**
(21) Numéro de dépôt: 17768496.6
(22) Date de dépôt: 05.09.2017
(51) Int. Cl.: A61M 1/02, A61M 1/36

(54) **UNITÉ DE FILTRATION COMPRENANT DES PORTIONS EN DÔME**
FILTRATIONSEINHEIT MIT KUPPELFÖRMIGEN ABSCHNITTEN
FILTRATION UNIT COMPRISING DOME-SHAPED PORTIONS

(30) Priorité: 08.09.2016 FR 1658378
(43) Date de publication de la demande: 17.07.2019
(73) Titulaire: Maco Pharma, 59420 Mouvaux (FR)
(72) Inventeur: BREBANT, Quentin, 59110 La Madeleine (FR); DEKERKE, Damien, 59710 Pont-à-Marc (FR)
(74) Mandataire: Sayettat, Julien Christian
(86) Numéro de dépôt international: PCT/FR2017/052352
(87) Numéro de publication internationale: WO 2018/046842

(56) Documents cités:
- EP-A2- 1 156 067
- US-A- 4 422 939
- US-A1- 2012 160 782

## Description

L'invention concerne une unité de filtration d'un fluide biologique tel que le sang ou un composant sanguin ainsi qu'un système à poches comprenant une telle unité de filtration.

L'invention s'applique au domaine de la filtration des fluides biologiques et notamment la filtration du sang ou des composants sanguins.

Dans le champ de la transfusion, il est classique de traiter le sang pour en éliminer les substances indésirables telles que les leucocytes, les pathogènes, certaines protéines - par exemple le prion -, et/ou les substances utilisées dans des procédés d'inactivation, de réduction et/ou d'élimination des pathogènes.

Pour éliminer ces substances indésirables par filtration, il existe deux grandes catégories d'unités de filtration : les unités de filtration dites souples, comme celle connue du document US2012/0160782 A1, comprenant une enveloppe extérieure souple, et les unités de filtration dites rigides, comme celle connue du document EP1156067, comprenant un boîtier rigide. Un milieu de filtration apte à éliminer les substances indésirables du fluide est enfermé dans ces unités de filtration.

Ces unités de filtration sont généralement intégrées dans des systèmes à poches permettant de traiter le sang et les composants sanguins en circuit clos.

Ainsi, après recueil du sang total, de tels systèmes à poches sont placés dans des appareils de centrifugation dans le but de séparer les différents composants sanguins en fonction de leur densité, en les soumettant à la force centrifuge.

Cependant, durant cette étape de centrifugation, il existe un risque non seulement que ces unités de filtration rigides soient endommagées mais encore que les unités de filtration rigides endommagent les poches adjacentes, rendant inutilisables les systèmes à poches et contaminant possiblement l'environnement extérieur et l'utilisateur.

En effet, comme illustré dans le document WO 2008/103142, les unités de filtration rigides comprennent des boîtiers aux bords formant des angles sensiblement droits et possèdent en outre des orifices d'entrée et/ou sortie de fluide faisant saillie du boîtier en formant des arêtes vives. Avec la pression générée par la centrifugation, ces parties anguleuses peuvent perforer les poches.

Pour éviter la dégradation des systèmes à poches durant la centrifugation, il a donc été développé des unités de filtration de type souple, par exemple comme celle du document EP-A-526 678. Ces unités de filtration souples sont cependant difficiles à fabriquer.

D'autre part, différentes solutions ont été proposées dans le but d'éviter les dommages liés aux unités de filtration rigides au cours de la centrifugation.

Par exemple, dans le document EP 0627 228, il est proposé de placer les unités de filtration en dehors du godet de centrifugation sur le rotor de la centrifugeuse.

Dans la même veine, dans le document US 5 100 564, l'unité de filtration est disposée au-dessus du godet de centrifugation à l'aide d'un support approprié.

D'autre part, le document US 4 422 939 décrit une unité de filtration pour le sang et la perfusion comprenant une coque supérieure et une coque inférieure. Chacune des coques comprend une surface plane et une partie pyramidale. La pyramide côté entrée permet une meilleure répartition du sang à filtrer et côté sortie sert de réservoir permettant d'égaliser la pression à l'intérieur de l'unité de filtration. Cette unité de filtration est notamment utilisée dans un circuit extracorporel lors d'interventions de cardiotomie et n'est donc pas prévue pour être centrifugée. Les pyramides comprennent en outre des arêtes vives qui rendent inapte à la centrifugation une telle unité de centrifugation.

L'invention propose une unité de filtration rigide qui peut être centrifugée dans un godet de centrifugation avec des poches souples sans les endommager.

Ainsi, et selon le premier aspect, l'invention propose une unité de filtration destinée à la filtration d'un fluide comprenant un boîtier rigide formé d'un premier élément de boîtier et d'un deuxième élément de boîtier entre lesquels un milieu de filtration est disposé de sorte à définir un compartiment d'entrée pour le fluide à filtrer et un compartiment de sortie pour le fluide filtré, ledit boîtier comportant un conduit d'entrée débouchant dans le compartiment d'entrée et un conduit de sortie débouchant dans le compartiment de sortie, chacun desdits éléments de boîtier comprenant un fond entouré par une paroi périphérique, le fond d'au moins un desdits éléments de boîtier comprenant une portion principale et au moins une portion en dôme qui est délimitée par un pourtour en présentant une paroi interne s'étendant depuis ledit pourtour vers un apex et une paroi externe s'étendant depuis ledit apex vers ledit pourtour, au moins un des conduits d'entrée et sortie débouchant au travers de la paroi externe, les parois interne et externe s'étendant respectivement dans un plan médian formant entre eux un angle strictement supérieur à 90°, l'apex présentant un profil extérieur courbe qui s'étend entre lesdits plans médians à la jonction entre les parois interne et externe.

Selon un deuxième aspect, l'invention concerne un système à poches pour la filtration d'un fluide, comprenant :
- une unité de filtration selon le premier aspect de l'invention, et
- une poche de recueil du filtrat, ladite poche de recueil étant reliée, par l'intermédiaire d'une première tubulure, au conduit de sortie de l'unité de filtration.

D'autres objets et avantages apparaîtront au cours de la description qui suit en lien avec les figures annexées selon lesquelles :
La figure 1 représente une vue schématique en perspective de l'unité de filtration selon une réalisation de l'invention.
Les figures 2 à 4 représentent des vues schématiques de trois profils différents de l'unité de filtration de la figure 1.
La figure 5 représente une vue schématique de dessus de l'unité de filtration de la figure 1.
La figure 6 représente une vue schématique en coupe et de profil de l'unité de filtration de la figure 1.
La figure 7 représente une vue schématique partielle, en coupe et de profil de l'unité de filtration de la figure 1.
La figure 8 représente une vue schématique en éclaté et de profil des premier et deuxième éléments de boîtier et du milieu de filtration de l'unité de filtration de la figure 1.
La figure 9 représente une vue schématique d'un système à poches comprenant l'unité de filtration de la figure 1.

L'invention concerne une unité de filtration destinée à la filtration d'un fluide, notamment du sang ou d'un composant sanguin tel qu'un plasma y compris un plasma riche en plaquettes et un plasma pauvre en plaquettes, un concentré de globules rouges ou un concentré de plaquettes sanguines.

L'unité de filtration est destinée à éliminer des composants cibles du fluide, notamment les leucocytes, les protéines prions ou les substances d'inactivation des pathogènes tel que le bleu de méthylène.

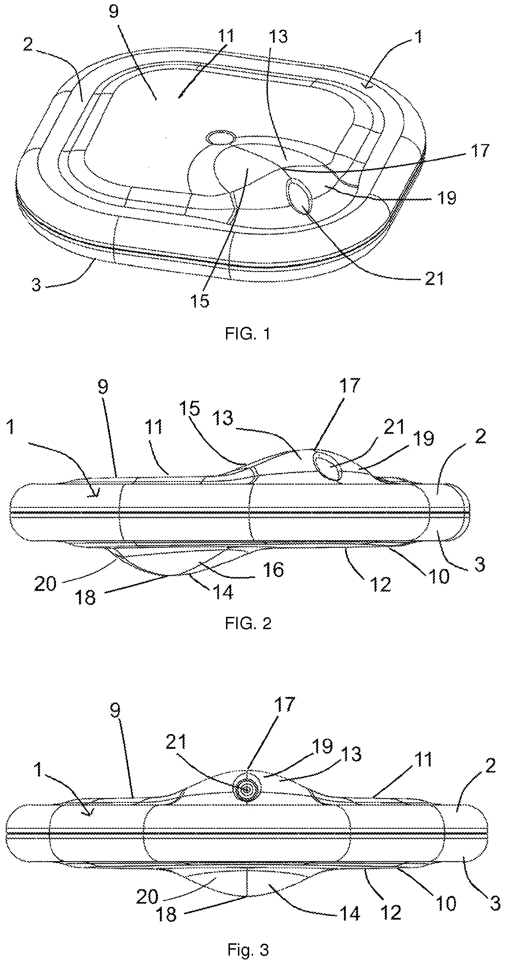

En relation avec les figures 1 à 8, l'unité de filtration 1 comprend un boîtier rigide formé d'un premier élément de boîtier 2 et d'un deuxième élément de boîtier 3 entre lesquels un milieu de filtration 4 est disposé de sorte à définir un compartiment d'entrée 5 pour le fluide à filtrer et un compartiment de sortie 6 pour le fluide filtré.

Les éléments de boîtier sont notamment réalisés dans un matériau polymère thermoplastique tel que le polycarbonate, polychlorure de vinyle, polypropylène ou acrylonitrile-butadiène-styrène.

Le compartiment d'entrée 5 est limité par le premier élément de boîtier 2 et le milieu de filtration 4. Le compartiment de sortie 6 est limité par le deuxième élément de boîtier 3 et le milieu de filtration 4.

Cette unité de filtration 1 est destinée à filtrer un fluide biologique qui est, par exemple un fluide biologique tel que le sang ou un composant sanguin.

Dans une réalisation, le milieu de filtration 4 est apte à éliminer les leucocytes du sang ou d'un composant sanguin. Le milieu de filtration comprend notamment une ou plusieurs couches d'un matériau tissé ou non-tissé. Par exemple, le milieu de filtration comprend un assemblage de plusieurs couches de non-tissé.

Le ou les matériau(x) formant la ou les couche(s) du milieu de déleucocytation sont choisi(s) dans le groupe comprenant les polymères ou les copolymères à base de polypropylène, de polyester, de polyamide, de polyéthylène haute ou basse densité, de polyuréthanne, de fluorure de polyvinylidène, de polyvinylpyrrolidone et leurs dérivés. Avantageusement, le matériau est du polypropylène, du polyéthylène téréphtalate ou du polybutylène téréphtalate.

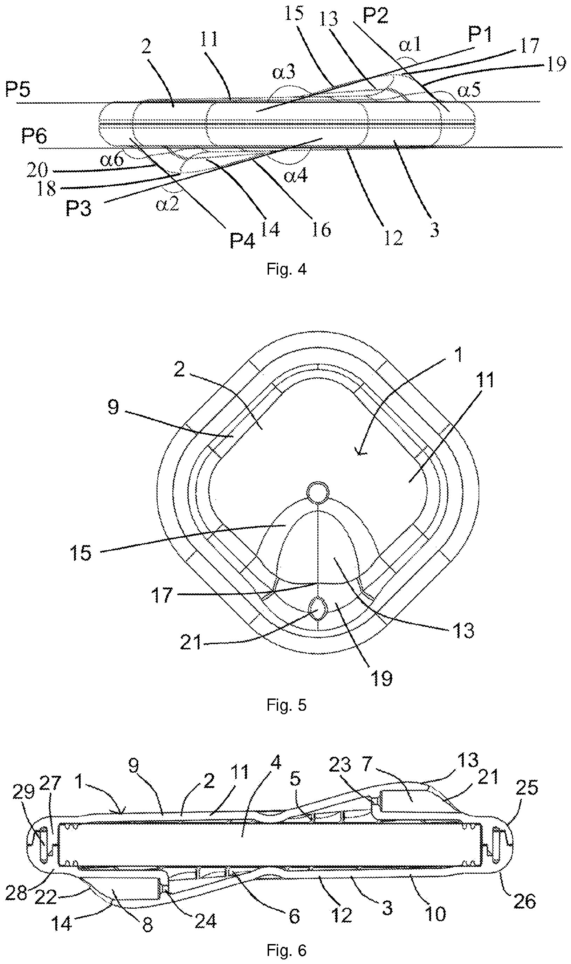

En relation avec la figure 6, le boîtier comporte un conduit d'entrée 7 débouchant dans le compartiment d'entrée 5 et un conduit de sortie 8 débouchant dans le compartiment de sortie 6.

Selon une réalisation, le conduit d'entrée 7 et le conduit de sortie 8 sont agencés parallèlement audit milieu de filtration 4.

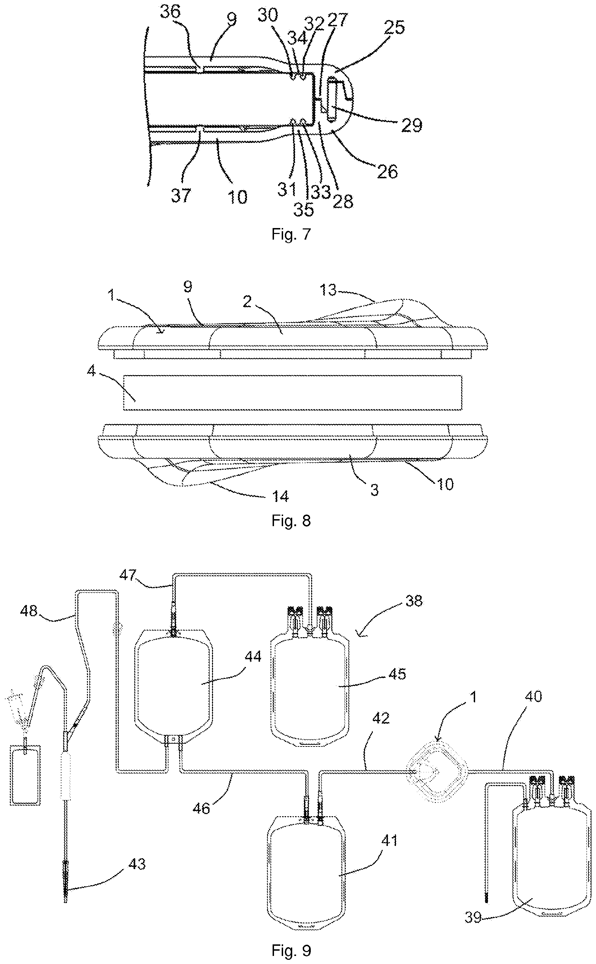

Les conduits d'entrée et sortie 7,8 sont notamment des conduits tubulaires creux et rigides. Ces conduits sont aptes à recevoir une tubulure souple pour pouvoir intégrer l'unité de filtration dans un système à poches tel que celui représenté sur la figure 9.

Dans le mode de réalisation représenté, le premier élément de boîtier 2 comprend le conduit d'entrée 7 pour le fluide à filtrer et le deuxième élément de boîtier 3 comprend le conduit de sortie 8 pour le filtrat.

Comme représenté sur les figures, chacun des premier et deuxième éléments 2,3 de boîtier comprend un fond 9,10 entouré par une paroi périphérique 25, 26, le fond d'au moins un desdits éléments de boîtier comprenant une portion principale 11,12 et au moins une portion en dôme 13,14 qui est délimitée par un pourtour en présentant une paroi interne 15,16 s'étendant depuis ledit pourtour vers un apex 17,18 et une paroi externe 19,20 s'étendant depuis ledit apex 17,18 vers ledit pourtour.

Dans la description et en relation avec l'unité de filtration, l'adjectif "interne" est à comprendre par opposition à l'adjectif "externe". Un moyen dit "externe" désigne un moyen qui est tourné à l'opposé de la partie centrale de l'unité de filtration.

Le fond 9,10 de chacun des éléments de boîtier s'étend dans un plan médian P5,P6 en étant entouré par une paroi périphérique 25, 26.

La portion principale 11,12 du fond des éléments de boîtier 2,3 est notamment sensiblement plane et définit un plan de base.

La portion en dôme 13,14 du fond des éléments de boîtier 2,3 ne comprend avantageusement aucune partie anguleuse, de sorte que le risque de perforer des poches adjacentes lors de la centrifugation est réduit.

Notamment et selon la figure 4, les parois interne 15 et externe 19 de la portion en dôme 13 de l'un des éléments de boîtier 2 s'étendent respectivement dans un plan médian P1,P2 formant entre eux un angle α1 strictement supérieur à 90°, particulièrement compris entre 100° et 130°.

Les parois interne 16 et externe 20 de la portion en dôme 14 de l'autre élément de boîtier 3 s'étendent respectivement dans un plan médian P3,P4 formant entre eux un angle α2 strictement supérieur à 90°, particulièrement compris entre 100° et 130°.

Par « plan médian » d'une paroi, on entend le plan situé au milieu de ladite paroi.

En outre, l'apex 17,18 de la portion en dôme 13,14 présente un profil extérieur courbe qui s'étend entre lesdits plans médians P1,P2 et P3,P4 à la jonction entre les parois interne 15,16 et externe 19,20.

Selon une réalisation, le profil extérieur courbe possède, vue de profil, un rayon de courbure strictement supérieure à la hauteur de la portion en dôme prise à partir du plan de base P5,P6 de la portion principale 11,12 du fond de l'élément de boîtier 2,3.

Comme illustré sur la figure 4, le plan médian de la paroi interne P1,P3 de la portion en dôme 13,14 forme un angle α3,α4 compris entre 120° et 150° avec le plan de base P5,P6 de la portion principale 11,12 du fond.

Le plan médian P2,P4 de la paroi externe 19,20 de la portion en dôme 13,14 forme un angle α5,α6 compris entre 120° et 150° avec un plan de base P5,P6 de la portion principale 11,12 du fond 9,10.

Ainsi, la portion en dôme 13,14 est relativement peu élevée de sorte que le volume mort du boîtier de l'unité de filtration 1 est réduit.

Plus particulièrement et en relation avec la figure 5, la paroi externe 19,20 s'étend depuis l'apex 17,18 vers une portion du pourtour qui est adjacente à la paroi périphérique 25, 26 du fond 9,10.

La paroi interne 15,16 s'étend vers l'apex 17,18 depuis une portion sensiblement en U du pourtour. Encore plus particulièrement, les extrémités des branches du U sont adjacentes à la paroi périphérique 25, 26 du fond 9,10.

Autrement dit, la portion en dôme 13,14 de l'élément de boîtier 2,3 est située au voisinage de la paroi périphérique 25, 26 du fond 9, 10 de l'élément de boîtier 2,3.

Selon une réalisation particulière, le fond 9,10 présente une dimension qui s'étend le long de la portion principale 11,12 et de la portion en dôme 13,14, la paroi interne 15,16 s'étendant le long de ladite dimension sur une distance comprise entre 20% et 40% de ladite dimension.

Comme représenté sur la figure 6, au moins un des conduits d'entrée 7 et sortie 8 débouche au travers de la paroi externe 19,20 de la portion en dôme 13,14 d'un élément de boîtier 2,3.

Plus particulièrement, une extrémité externe d'un conduit d'entrée 7 et sortie 8 débouche dans la paroi externe 19,20 en étant adjacente à la paroi périphérique 25, 26 du fond 9,10 de l'élément de boîtier 2,3.

Avantageusement, la paroi externe 19,20 présente un orifice dans lequel au moins une extrémité externe 21,22 d'un conduit d'entrée 7 et sortie 8 est inscrite. La portion en dôme 13,14 recouvre alors entièrement le conduit d'entrée 7 ou sortie 8, de sorte qu'aucun élément saillant fragile ne s'étend de la portion en dôme 13,14.

Les parties en relief du boîtier présentent ainsi une forme courbe de sorte que, quelle que soit la position du boîtier dans le godet de centrifugation, le risque de rupture des conduits d'entrée et de sortie 7,8 et le risque d'endommager les poches avoisinantes sont réduits et même éliminés.

Et, dans cette réalisation, l'extrémité externe 21,22 du conduit d'entrée et/ou sortie 7,8 n'est pas droite, c'est-à-dire qu'elle n'est pas perpendiculaire à l'axe longitudinal du conduit d'entrée et/ou sortie 7,8.

Par exemple, comme représenté sur la figure 5, l'extrémité externe du conduit d'entrée et/ou sortie 7,8 présente un biseau. Dans le cas d'une conduite tubulaire, l'orifice est alors sensiblement ovale.

Selon une réalisation particulière représentée sur la figure 6, l'un au moins des conduits d'entrée et de sortie 7,8 présente à son extrémité interne 23,24 une ouverture ayant une section plus petite que la section de l'ouverture dudit conduit 7,8 à son extrémité externe 21,22 (figure 6).

Afin de permettre une meilleure vidange de l'unité de filtration 1, l'extrémité interne 24 du conduit de sortie 8 est pourvue d'une restriction de flux formée par une réduction de section par rapport à la section de ce conduit à son extrémité externe. Par effet Venturi, la restriction de flux créé une accélération du débit de fluide filtré qui tend à aspirer le fluide en dehors de l'unité de filtration 1.

En particulier, l'extrémité interne 23 du conduit d'entrée 7 est également pourvue d'une restriction de flux. Encore plus particulièrement, le conduit d'entrée 7 et le conduit de sortie 8 présentent sensiblement la même géométrie et les mêmes dimensions.

Avantageusement, chacun des éléments de boîtier 2,3 présente une portion en dôme 13, le conduit d'entrée 7 débouchant dans la paroi externe 19 de la portion en dôme 13 dudit premier élément de boîtier 2 et le conduit de sortie 8 débouchant dans la paroi externe 20 de la portion en dôme 14 dudit deuxième élément de boîtier 3.

Selon une réalisation, la portion en dôme 13 du premier élément de boîtier 2 et la portion en dôme 14 du deuxième élément de boîtier 3 présentent sensiblement la même géométrie et les mêmes dimensions.

En relation avec les figures 6 et 7, chacun des premier et deuxième éléments de boîtier 2,3 comprend une cavité formée du fond 9,10 et d'une paroi latérale périphérique 25,26, la portion en dôme 13,14 étant agencée sur la surface extérieure du fond 9,10 de l'un des premier ou deuxième éléments de boîtier 2,3.

En particulier, la surface extérieure de l'une ou des deux parois latérales périphériques 25,26 sont courbes. Avantageusement, comme illustré sur les figures 5 et 6, les deux parois latérales périphériques 25,26 formant ensemble le bord périphérique du boîtier de l'unité de filtration présentent un profil extérieur courbe qui s'étend entre les plans médians P5,P6 des fonds 9,10 du premier et deuxième élément de boîtier. Ainsi, le boîtier de l'unité de filtration 1 avec ses portions en dôme 13,14 et ses parois latérales courbes 25,26 ne comprend aucune saillie anguleuse ou arête vive susceptible d'endommager les poches pendant la centrifugation.

Notamment, le profil extérieur courbe du bord périphérique présente un rayon de courbure qui est supérieur à la moitié de la distance entre les deux plans médians P5,P6.

Le rayon de courbure est le rayon du cercle tangent au profil extérieur courbe du bord périphérique vu en section transversale.

Selon une première réalisation représentée sur les figures, les périphéries des éléments de boîtier présentent une géométrie complémentaire qui est agencée pour former le bord périphérique ayant un profil extérieur courbe.

En variante non représentée, le boîtier de l'unité de filtration 1 comprend en outre un ou plusieurs éléments protecteurs rapportés par surmoulage d'un matériau thermoplastique élastomère configurés pour protéger le boîtier des chocs et/ou éviter d'endommager les poches souples.

Le premier élément de boîtier 2 et le deuxième élément de boîtier 3 sont assemblés de façon étanche sur leur périphérie, de sorte à former un boîtier étanche au fluide.

Par exemple, les premier et deuxième éléments de boîtier 2,3 sont assemblés entre eux par soudure par haute fréquence, par radiofréquence ou par ultrasons. En variante, les éléments de boîtier 2,3 sont assemblés par collage.

Afin de faciliter l'assemblage des deux éléments de boîtier 2,3, les éléments de boîtier 2,3 sont ajustés entre eux par assemblage de forme ou par encliquetage.

En relation avec les figures 6 et 7, la paroi latérale périphérique 25 du premier élément de boîtier 2 et la paroi latérale périphérique 26 du deuxième élément de boîtier 3 sont mises en contact sur leur surface extrême par conjugaison de forme.

De plus, chacun des éléments de boîtier 2,3 comprend en outre un élément intérieur de conjugaison 27,28 sensiblement parallèle à la paroi latérale 25,26. Les deux éléments intérieurs de conjugaison 27,28 de chacun des éléments de boîtier 2,3 coopèrent par conjugaison de forme, de sorte qu'une fois les éléments de boîtier 2,3 assemblés entre eux, une partie creuse 29 est formée entre les parois latérales 25,26 et éléments intérieurs de conjugaison 27,28.

Le milieu de filtration 4 est disposé entre les deux éléments de boîtier 2,3 et y est maintenu serré.

Dans une réalisation particulière et afin d'assurer une étanchéité au fluide entre le compartiment d'entrée 5 et le compartiment de sortie 6 de l'unité de filtration 1, chacun des éléments de boîtier 2,3 comprend une saillie intérieure 30,31 agencée pour comprimer le milieu de filtration 4 le long de sa périphérie.

Les saillies intérieures 30,31 sont agencées pour comprimer le milieu de filtration 4 le long de sa périphérie de sorte à créer une zone intérieure d'étanchéité au fluide.

Avantageusement, chacun des éléments de boîtier 2,3 comprend en outre une saillie extérieure 32,33 agencée pour comprimer le milieu de filtration 4 le long de sa périphérie.

Les saillies extérieures 32,33 sont agencées pour comprimer le milieu de filtration 4 de sorte à créer une zone extérieure d'étanchéité au fluide.

Dans la description et en relation avec l'unité de filtration, l'adjectif "extérieur" est à comprendre par opposition à l'adjectif "intérieur". Un moyen dit "intérieur" désigne un moyen situé dans une zone de l'unité de filtration plus proche du centre de l'unité de filtration qu'un moyen dit "extérieur".

Ainsi, si le fluide n'est pas arrêté ou suffisamment ralenti par le premier couple des saillies intérieures 30,31, le couple des saillies extérieures 32,33 fournit un deuxième passage rétréci, suffisant pour éviter le contournement du milieu de filtration 4 par le fluide.

Ceci peut être le cas si, par exemple, un défaut ou une irrégularité dans le milieu de filtration 4 se situe au niveau des saillies intérieures 30,31, empêchant le milieu de filtration 4 d'être suffisamment comprimé pour créer une étanchéité au fluide.

Par exemple, sur la figure 6, les saillies intérieures 30,31 et extérieures 32,33 sont formées de nervures continues. Ces nervures présentent une section de forme essentiellement triangulaire, à sommet arrondi.

En relation avec les éléments de boîtier 2,3, ceux-ci comprennent notamment une première et une deuxième partie de serrage 34,35, respectivement agencées pour maintenir serré dans le boîtier ledit milieu de filtration le long de sa périphérie.

Les parties de serrage 34,35 des éléments de boîtier compriment le milieu de filtration 4 de manière à en réduire l'épaisseur, sans pour autant pouvoir assurer une étanchéité au fluide.

Notamment, les parties de serrage 34,35 sont agencées sur le fond 9,10 des cavités des éléments de boîtiers 2,3.

Plus particulièrement et comme représenté sur la figure 7, le premier et le deuxième élément de boîtier 2,3 comprennent respectivement une première et une deuxième cavité pourvues chacune d'une plate-forme périphérique dirigée vers l'extérieur formant la première et deuxième partie de serrage 34,35 respectivement, agencées pour maintenir serré dans le boîtier le milieu de filtration 4 le long de sa périphérie.

Dans cette configuration, le milieu de filtration 4 est maintenu serré entre la première et la deuxième plate-forme, ce qui permet de compresser le milieu de filtration le long de sa périphérie. Les saillies intérieures 30,31 et extérieures 32,33 sont alors situées sur ces plates-formes, notamment à leur extrémité intérieure.

La paroi latérale 25,26 de chacun des éléments de boîtier 2,3 présentant une surface extérieure courbe, s'étend à partir de la plate-forme périphérique de chacune des cavités des éléments de boîtier.

Selon une forme particulière de réalisation, le premier élément de boîtier 2 et le deuxième élément de boîtier 3 comprennent chacun sur leur surface intérieure, en particulier sur la surface intérieure du fond 9,10, un déflecteur de flux formé d'une pluralité de reliefs 36,37.

En relation avec la figure 7, chacun des éléments de boîtier 2,3 est pourvu sur la surface intérieure du fond 9,10 d'une pluralité de reliefs 36,37 linéaires dirigés obliquement vers le conduit d'entrée et de sortie. Du côté du compartiment d'entrée 5, les déflecteurs aident à répartir le flux de fluide à filtrer sur toute la surface du milieu de filtration. Du côté du compartiment de sortie 6, les déflecteurs aident à diriger le fluide filtré vers le conduit de sortie de l'unité de filtration.

Selon un deuxième aspect et en relation avec la figure 9, l'invention concerne un système à poches 38 pour la filtration d'un fluide, comprenant :
- une unité de filtration 1 selon le premier aspect de l'invention, et
- une poche de recueil du filtrat 39, ladite poche de recueil du filtrat 39 étant reliée, par l'intermédiaire d'une première tubulure 40, au conduit de sortie 8 de l'unité de filtration 1.

Pour introduire le fluide à filtrer, le système à poches 38 comprend en outre une poche 41 destinée à contenir le fluide à filtrer, ladite poche 41 pour le fluide à filtrer étant reliée par l'intermédiaire d'une deuxième tubulure 42, au conduit d'entrée 7 de l'unité de filtration 1.

Cette poche 41 pour le fluide à filtrer est soit connectée de fabrication à l'unité de filtration 1 par l'intermédiaire de la deuxième tubulure 42, soit connectée par l'utilisateur à l'aide d'un connecteur de type perforateur pourvue à l'extrémité libre de la deuxième tubulure (non représentée).

La figure 9 représente plus particulièrement un exemple d'un système à poches 38 utilisé pour séparer en plasma et concentré de globules rouges un don de sang total et pour déleucocyter le concentré de globules rouges.

Le système à poches 38 comprend une aiguille 43 pour prélever le sang d'un donneur, une poche primaire 44 destinée à recueillir du sang total, une poche satellite 45 destinée à recevoir le plasma en communication fluidique avec la poche primaire, une poche satellite 41 destinée à recevoir le concentré de globules rouges en communication fluidique avec la poche primaire, une unité de filtration 1 telle que décrite précédemment destinée à filtrer les leucocytes du concentré de globules rouges en communication fluidique avec la poche satellite pour recevoir le concentré de globules rouges, et une poche de recueil du filtrat 39 en communication fluidique avec l'unité de filtration 1. Les poches et l'unité de filtration 1 sont reliées entre elles au moyen de tubulures souples 40,42,46,47,48 et stérilisables.

La poche primaire 44 contient un anticoagulant de type CPD et la poche de recueil du filtrat 39 contient une solution additive pour la conservation des globules rouges de type SAGM (Saline Adénine Glucose Mannitol).

En utilisation, le système à poches 38 contenant le sang total mélangé avec la solution anticoagulante dans la poche primaire 44 est placé dans un godet de centrifugation pour être centrifugé de sorte à séparer le sang en trois couches : un surnageant comprenant le plasma, une couche intermédiaire dite byffy coat comprenant un mélange de plaquettes, plasma et globules rouges et un culot comprenant le concentré de globules rouges.

Par pression sur la poche primaire 44, le plasma est envoyé dans la poche satellite 45 destinée à recevoir le plasma et le concentré de globules rouges est envoyé dans la poche satellite 41 destinée à recevoir les globules rouges. Le buffy-coat reste dans la poche primaire 44.

La solution additive est ensuite envoyée de la poche 39 de recueil du filtrat dans la poche satellite 41 destinée à recevoir les globules rouges. Le concentré de globules rouges, mélangé avec la solution additive, est finalement filtré par gravité au travers de l'unité de filtration 1 et le filtrat est reçu dans la poche destinée à recueillir le filtrat 39.

## Revendications

1. Unité de filtration (1) destinée à la filtration d'un fluide comprenant un boîtier rigide formé d'un premier élément de boîtier (2) et d'un deuxième élément de boîtier (3) entre lesquels un milieu de filtration (4) est disposé de sorte à définir un compartiment d'entrée (5) pour le fluide à filtrer et un compartiment de sortie (6) pour le fluide filtré, ledit boîtier comportant un conduit d'entrée (7) débouchant dans le compartiment d'entrée (5) et un conduit de sortie (8) débouchant dans le compartiment de sortie (6), chacun desdits éléments de boîtier (2,3) comprenant un fond (9,10) entouré par une paroi latérale périphérique (25, 26), le fond (9,10) d'au moins un desdits éléments de boîtier comprenant une portion principale (11,12) et au moins une portion en dôme (13,14) qui est délimitée par un pourtour en présentant une paroi interne (15,16) s'étendant depuis ledit pourtour vers un apex (17,18) et une paroi externe (19,20) s'étendant depuis ledit apex (17,18) vers ledit pourtour, au moins un des conduits d'entrée et sortie (7,8) débouchant au travers de la paroi externe (19,20), ladite unité de filtration étant **caractérisée en ce que** les parois interne (15,16) et externe (19,20) s'étendent respectivement dans un plan médian (P1,P2,P3,P4) formant entre eux un angle (α1,α2) strictement supérieur à 90°, l'apex (17,18) présentant un profil extérieur courbe qui s'étend entre lesdits plans médians à la jonction entre les parois interne (15,16) et externe (19,20).

2. Unité de filtration selon la revendication 1, **caractérisée en ce que** les plans médians (P1,P2,P3,P4) forment entre eux un angle (α1,α2) compris entre 100° et 130°.

3. Unité de filtration selon l'une des revendications 1 ou 2, **caractérisée en ce que** le plan médian (P1,P3) de la paroi interne (15,16) forme un angle (α3,α4) compris entre 120° et 150° avec un plan de base (P5,P6) de la portion principale (11,12) du fond (9,10).

4. Unité de filtration selon l'une quelconque des revendications 1 à 3, **caractérisée en ce que** le plan médian (P2,P4) de la paroi externe forme un angle (α5,α6) compris entre 120° et 150° avec un plan de base (P5 ,P6) de la portion principale (11,12) du fond (9,10).

5. Unité de filtration selon l'une quelconque des revendications 1 à 4, **caractérisée en ce que** la paroi externe (19,20) s'étend depuis l'apex (17,18) vers une portion du pourtour qui est adjacente à la paroi périphérique (25, 26).

6. Unité de filtration selon l'une quelconque des revendications 1 à 5, **caractérisée en ce que** la paroi interne (15,16) s'étend vers l'apex (17,18) depuis une portion sensiblement en U du pourtour.

7. Unité de filtration selon la revendication 6, **caractérisée en ce que** les extrémités des branches du U sont adjacentes à la paroi périphérique (25, 26).

8. Unité de filtration selon l'une quelconque des revendications 1 à 7, **caractérisée en ce que** le fond (9,10) présente une dimension qui s'étend le long de la portion principale (11,12) et de la portion en dôme (13,14), la paroi interne (15,16) s'étendant le long de ladite dimension sur une distance comprise entre 20% et 40% de ladite dimension.

9. Unité de filtration selon l'une quelconque des revendications 1 à 8, **caractérisée en ce que** la paroi externe (19,20) présente un orifice dans lequel au moins une extrémité externe (21,22) d'un conduit d'entrée et sortie (7,8) est inscrite.

10. Unité de filtration selon l'une quelconque des revendications 1 à 9, **caractérisée en ce qu'**au moins une extrémité externe (21,22) d'un conduit d'entrée et sortie (7,8) débouche dans la paroi externe (19,20) en étant adjacente à la paroi périphérique (25, 26).

11. Unité de filtration selon l'une quelconque des revendications 1 à 10, **caractérisée en ce que** chacun des éléments de boîtier (2,3) présente une portion en dôme (13,14), le conduit d'entrée (7) débouchant dans la paroi externe (19) de la portion en dôme (13) dudit premier élément de boîtier (2) et le conduit de sortie (8) débouchant dans la paroi externe (20) de la portion en dôme (14) dudit deuxième élément de boîtier (3).

12. Unité de filtration selon l'une quelconque des revendications 1 à 11, **caractérisée en ce que** le conduit d'entrée (7) et le conduit de sortie (8) sont agencés parallèlement au milieu de filtration (4).

13. Unité de filtration selon l'une quelconque des revendications 1 à 12, **caractérisée en ce que** l'un au moins des conduits d'entrée et de sortie (7,8) présente à son extrémité interne (23,24) une ouverture ayant une section plus petite que la section de l'ouverture dudit conduit (7,8) à son extrémité externe (21,22).

14. Unité de filtration selon l'une quelconque des revendications 1 à 13, **caractérisée en ce que** chacun des éléments de boîtier (2,3) comprend une saillie intérieure (30,31) agencée pour comprimer le milieu de filtration (4) le long de sa périphérie.

15. Unité de filtration selon la revendication 14, **caractérisée en ce que** chacun des éléments de boîtier (2,3) comprend en outre une saillie extérieure (32,33) agencée pour comprimer le milieu de filtration (4) le long de sa périphérie.

16. Unité de filtration selon l'une quelconque des revendications 1 à 15, **caractérisée en ce que** le premier élément de boîtier (2) et le deuxième élément de boîtier (3) comprennent chacun sur leur surface intérieure un déflecteur de flux formé d'une pluralité de reliefs (36,37).

17. Unité de filtration selon l'une quelconque des revendications 1 à 16, **caractérisée en ce que** les éléments de boîtiers (2,3) sont assemblés entre eux de façon étanche au niveau de leur périphérie.

18. Unité de filtration selon l'une quelconque des revendications 1 à 17, **caractérisée en ce que** le milieu de filtration (4) comprend un assemblage de couches de non-tissé.

19. Unité de filtration selon l'une quelconque des revendications 1 à 18, **caractérisée en ce que** le milieu de filtration (4) est apte à éliminer les leucocytes du sang ou d'un composant sanguin.

20. Système à poches (38) pour la filtration d'un fluide, **caractérisé en ce qu'**il comprend :
- une unité de filtration (1) selon l'une quelconque des revendications 1 à 19, et
- une poche de recueil du filtrat (39), ladite poche de recueil du filtrat (39) étant reliée, par l'intermédiaire d'une première tubulure (40), au conduit de sortie (8) de l'unité de filtration (1).

21. Système à poches selon la revendication 20, **caractérisé en ce qu'**il comprend en outre une poche (41) destinée à contenir le fluide à filtrer, ladite poche (41) destinée à contenir le fluide à filtrer étant reliée par l'intermédiaire d'une deuxième tubulure (42) au conduit d'entrée (7) de l'unité de filtration (1).

## Patentansprüche

1. Filtrationseinheit (1), die zur Filtration eines Fluids bestimmt ist, umfassend ein starres Gehäuse, das von einem ersten Gehäuseelement (2) und einem zweiten Gehäuseelement (3) gebildet wird, zwischen denen ein Filtermedium (4) angeordnet ist, sodass ein Eintrittsfach (5) für das zu filtrierende Fluid und ein Austrittsfach (6) für das filtrierte Fluid definiert werden, wobei das Gehäuse einen Eintrittskanal (7), der in das Eintrittsfach (5) mündet, und einen Austrittskanal (8), der in das Austrittsfach (6) mündet, umfasst, wobei jedes der Gehäuseelemente (2, 3) einen Boden (9, 10) umfasst, der von einer umlaufenden Seitenwand (25, 26) umgeben ist, wobei der Boden (9, 10) mindestens eines der Gehäuseelemente einen Hauptabschnitt (11, 12) und mindestens einen kuppelförmigen Abschnitt (13, 14) umfasst, der von einer Umrandung begrenzt ist, indem er eine Innenwand (15, 16), die sich von der Umrandung zu einer Spitze (17, 18) erstreckt, und eine Außenwand (19, 20), die sich von der Spitze (17, 18) zu der Umrandung erstreckt, aufweist, wobei mindestens einer des Eintritts- und Austrittskanals (7, 8) durch die Außenwand (19, 20) mündet, wobei die Filtrationseinheit **dadurch gekennzeichnet ist, dass** sich die Innen- (15, 16) und Außenwand (19, 20) jeweils in einer Mittelebene (P1, P2, P3, P4) erstrecken, die dazwischen einen Winkel (α1, α2) bilden, der streng über 90° beträgt, wobei die Spitze (17, 18) ein gekrümmtes Außenprofil aufweist, das sich zwischen den Mittelebenen an der Schnittstelle zwischen der Innen- (15, 16) und Außenwand (19, 20) erstreckt.

2. Filtrationseinheit nach Anspruch 1, **dadurch gekennzeichnet, dass** die Mittelebenen (P1, P2, P3, P4) dazwischen einen Winkel (α1, α2) bilden, der zwischen 100° und 130° beträgt.

3. Filtrationseinheit nach einem der Ansprüche 1 oder 2, **dadurch gekennzeichnet, dass** die Mittelebene (P1, P3) der Innenwand (15, 16) einen Winkel (α3, α4), der zwischen 120° und 150° beträgt, mit einer Basisebene (P5, P6) des Hauptabschnitts (11, 12) des Bodens (9, 10) bildet.

4. Filtrationseinheit nach einem der Ansprüche 1 bis 3, **dadurch gekennzeichnet, dass** die Mittelebene (P2, P4) der Außenwand einen Winkel (α5, α6), der zwischen 120° und 150° beträgt, mit einer Basisebene (P5, P6) des Hauptabschnitts (11, 12) des Bodens (9, 10) bildet.

5. Filtrationseinheit nach einem der Ansprüche 1 bis 4, **dadurch gekennzeichnet, dass** sich die Außenwand (19, 20) von der Spitze (17, 18) zu einem Abschnitt der Umrandung erstreckt, der an die umlaufende Wand (25, 26) angrenzt.

6. Filtrationseinheit nach einem der Ansprüche 1 bis 5, **dadurch gekennzeichnet, dass** sich die Innenwand (15, 16) zu der Spitze (17, 18) ausgehend von einem im Wesentlichen U-förmigen Abschnitt der Umrandung erstreckt.

7. Filtrationseinheit nach Anspruch 6, **dadurch gekennzeichnet, dass** die Enden der Schenkel des U an die umlaufende Wand (25, 26) angrenzen.

8. Filtrationseinheit nach einem der Ansprüche 1 bis 7, **dadurch gekennzeichnet, dass** der Boden (9, 10) eine Abmessung aufweist, die sich entlang des Hauptabschnitts (11, 12) und des kuppelförmigen Abschnitts (13, 14) erstreckt, wobei sich die Innenwand (15, 16) entlang der Abmessung auf einer Distanz erstreckt, die zwischen 20 % und 40 % der Abmessung beträgt.

9. Filtrationseinheit nach einem der Ansprüche 1 bis 8, **dadurch gekennzeichnet, dass** die Außenwand (19, 20) eine Öffnung aufweist, in die sich mindestens ein Außenende (21, 22) eines Eintritts- und Austrittskanals (7, 8) einfügt.

10. Filtrationseinheit nach einem der Ansprüche 1 bis 9, **dadurch gekennzeichnet, dass** mindestens ein Außenende (21, 22) eines Eintritts- und Austrittskanals (7, 8) in die Außenwand (19, 20) mündet, indem es an die umlaufende Wand (25, 26) angrenzt.

11. Filtrationseinheit nach einem der Ansprüche 1 bis 10, **dadurch gekennzeichnet, dass** jedes der Gehäuseelemente (2, 3) einen kuppelförmigen Abschnitt (13, 14) aufweist, wobei der Eintrittskanal (7) in die Außenwand (19) des kuppelförmigen Abschnitts (13) des ersten Gehäuseelements (2) mündet und der Austrittskanal (8) in die Außenwand (20) des kuppelförmigen Abschnitts (14) des zweiten Gehäuseelements (3) mündet.

12. Filtrationseinheit nach einem der Ansprüche 1 bis 11, **dadurch gekennzeichnet, dass** der Eintrittskanal (7) und der Austrittskanal (8) parallel zum Filtermedium (4) angeordnet sind.

13. Filtrationseinheit nach einem der Ansprüche 1 bis 12, **dadurch gekennzeichnet, dass** mindestens einer des Eintritts- und Austrittskanals (7, 8) an seinem Innenende (23, 24) eine Öffnung aufweist, die einen kleineren Querschnitt aufweist als der Querschnitt der Öffnung des Kanals (7, 8) an seinem Außenende (21, 22).

14. Filtrationseinheit nach einem der Ansprüche 1 bis 13, **dadurch gekennzeichnet, dass** jedes der Gehäuseelemente (2, 3) einen Innenvorsprung (30, 31) umfasst, der angeordnet ist, um das Filtermedium (4) entlang seiner Peripherie zu komprimieren.

15. Filtrationseinheit nach Anspruch 14, **dadurch gekennzeichnet, dass** jedes der Gehäuseelemente (2, 3) des Weiteren einen Außenvorsprung (32, 33) umfasst, der angeordnet ist, um das Filtermedium (4) entlang seiner Peripherie zu komprimieren.

16. Filtrationseinheit nach einem der Ansprüche 1 bis 15, **dadurch gekennzeichnet, dass** das erste Gehäuseelement (2) und das zweite Gehäuseelement (3) jeweils auf ihrer Innenfläche einen Strömungsabweiser umfassen, der von einer Vielzahl von Erhebungen (36, 37) gebildet wird.

17. Filtrationseinheit nach einem der Ansprüche 1 bis 16, **dadurch gekennzeichnet, dass** die Gehäuseelemente (2, 3) auf Höhe ihrer Peripherie dicht zusammengefügt sind.

18. Filtrationseinheit nach einem der Ansprüche 1 bis 17, **dadurch gekennzeichnet, dass** das Filtermedium (4) einen Schichtaufbau aus Vliesstoff umfasst.

19. Filtrationseinheit nach einem der Ansprüche 1 bis 18, **dadurch gekennzeichnet, dass** das Filtermedium (4) fähig ist, die Leukozyten aus dem Blut oder aus einem Blutbestandteil zu eliminieren.

20. Beutelsystem (38) für die Filtration eines Fluids, **dadurch gekennzeichnet, dass** es umfasst:
- eine Filtrationseinheit (1) nach einem der Ansprüche 1 bis 19 und
- einen Filtratsammelbeutel (39), wobei der Filtratsammelbeutel (39) mittels einer ersten Leitung (40) mit dem Austrittskanal (8) der Filtrationseinheit (1) verbunden ist.

21. Beutelsystem nach Anspruch 20, **dadurch gekennzeichnet, dass** es des Weiteren einen Beutel (41) umfasst, der dazu bestimmt ist, das zu filtrierende Fluid zu enthalten, wobei der Beutel (41), der dazu bestimmt ist, das zu filtrierende Fluid zu enthalten, mittels einer zweiten Leitung (42) mit dem Eintrittskanal (7) der Filtrationseinheit (1) verbunden ist.

## Claims

1. Filtration unit (1) intended for filtering a fluid comprising a rigid housing formed of a first housing element (2) and a second housing element (3) between which a filtration medium (4) is disposed so as to define an inlet compartment (5) for the fluid to be filtered and an outlet compartment (6) for the filtered fluid, said housing including an inlet conduit (7) opening onto the inlet compartment (5) and an outlet conduit (8) opening onto the outlet compartment (6), each of said housing elements (2, 3) comprising a base (9, 10) surrounded by a peripheral side wall (25, 26), the base (9, 10) of at least one of said housing elements comprising a main portion (11, 12) and at least one domed portion (13, 14) which is delimited by a perimeter having an inner wall (15, 16) extending from said perimeter to an apex (17, 18) and an outer wall (19, 20) extending from said apex (17, 18) to said perimeter, at least one of the inlet and outlet conduits (7, 8) opening out through the outer wall (19, 20), said filtration unit being **characterised in that** the inner (15, 16) and outer (19, 20) walls extend respectively in a median plane (P1, P2, P3, P4) forming therebetween an angle (α1, α2) strictly greater than 90°, the apex (17, 18) having a curved outer profile which extends between said median planes at the junction between the inner (15, 16) and outer (19, 20) walls.

2. Filtration unit according to claim 1, **characterised in that** the median planes (P1, P2, P3, P4) form therebetween an angle (α1, α2) between 100° and 130°.

3. Filtration unit according to one of claims 1 or 2, **characterised in that** the median plane (P1, P3) of the inner wall (15, 16) forms an angle (α3, α4) between 120° and 150° with a base plane (P5, P6) of the main portion (11, 12) of the base (9, 10).

4. Filtration unit according to any one of claims 1 to 3, **characterised in that** the median plane (P2, P4) of the outer wall forms an angle (α5, α6) between 120° and 150° with a base plane (P5, P6) of the main portion (11, 12) of the base (9, 10).

5. Filtration unit according to any one of claims 1 to 4, **characterised in that** the outer wall (19, 20) extends from the apex (17, 18) to a portion of the perimeter which is adjacent to the peripheral wall (25, 26).

6. Filtration unit according to any one of claims 1 to 5, **characterised in that** the inner wall (15, 16) extends to the apex (17, 18) from a substantially U-shaped portion of the perimeter.

7. Filtration unit according to claim 6, **characterised in that** the ends of the arms of the U are adjacent to the peripheral wall (25, 26).

8. Filtration unit according to any one of claims 1 to 7, **characterised in that** the base (9, 10) has a dimension that extends along the main portion (11, 12) and the domed portion (13, 14), the inner wall (15, 16) extending along said dimension over a distance between 20% and 40% of said dimension.

9. Filtration unit according to any one of claims 1 to 8, **characterised in that** the outer wall (19, 20) has an orifice wherein at least an outer end (21, 22) of an inlet and outlet conduit (7, 8) is inscribed.

10. Filtration unit according to any one of claims 1 to 9, **characterised in that** at least one outer end (21, 22) of an inlet and outlet conduit (7, 8) opens into the outer wall (19, 20) while being adjacent to the peripheral wall (25, 26).

11. Filtration unit according to any one of claims 1 to 10, **characterised in that** each of the housing elements (2, 3) has a domed portion (13, 14), the inlet conduit (7) opening into the outer wall (19) of the domed portion (13) of said first housing element (2) and the outlet conduit (8) opening into the outer wall (20) of the domed portion (14) of said second housing element (3).

12. Filtration unit according to any one of claims 1 to 11, **characterised in that** the inlet conduit (7) and the outlet conduit (8) are arranged parallel with the filtration medium (4).

13. Filtration unit according to any one of claims 1 to 12, **characterised in that** one at least of the inlet and outlet conduits (7, 8) has at the inner end (23, 24) thereof an opening having a smaller cross-section than the cross-section of the opening of said conduit (7, 8) at the outer end (21, 22) thereof.

14. Filtration unit according to any one of claims 1 to 13, **characterised in that** each of the housing elements (2, 3) comprises an internal projection (30, 31) arranged to compress the filtration medium (4) along the periphery thereof.

15. Filtration unit according to claim 14, **characterised in that** each of the housing elements (2, 3) further comprises an external projection (32, 33) arranged to compress the filtration medium (4) along the periphery thereof.

16. Filtration unit according to any one of claims 1 to 15, **characterised in that** the first housing element (2) and the second housing element (3) each comprise on the internal surface thereof a flow deflector formed of a plurality of reliefs (36, 37).

17. Filtration unit according to any one of claims 1 to 16, **characterised in that** the housing elements (2, 3) are assembled together tightly at the level of the periphery thereof.

18. Filtration unit according to any one of claims 1 to 17, **characterised in that** the filtration medium (4) comprises an assembly of nonwoven layers.

19. Filtration unit according to any one of claims 1 to 18, **characterised in that** the filtration medium (4) is suitable for removing leukocytes from blood or from a blood component.

20. Bag system (38) for filtering a fluid, **characterised in that** it comprises:
- a filtration unit (1) according to any one of claims 1 to 19, and
- a bag for collecting the filtrate (39), said bag for collecting the filtrate (39) being connected, via a first tube (40), to the outlet conduit (8) of the filtration unit (1).

21. Bag system according to claim 20, **characterised in that** it further comprises a bag (41) intended to contain the fluid to be filtered, said bag (41) intended to contain the fluid to be filtered being connected via a second tube (42) to the inlet conduit (7) of the filtration unit (1).
